(19) Europäisches Patentamt
European Patent Office
Office européen des brevets

(11) EP 1 321 127 A2

(12) **DEMANDE DE BREVET EUROPEEN**

(43) Date de publication:
**25.06.2003 Bulletin 2003/26**

(51) Int Cl.$^7$: **A61K 7/06**, A61K 7/02

(21) Numéro de dépôt: **03290733.9**

(22) Date de dépôt: **28.08.2000**

(84) Etats contractants désignés:
**AT BE CH CY DE DK ES FI FR GB GR IE IT LI LU MC NL PT SE**

(30) Priorité: **16.09.1999 FR 9911593**

(62) Numéro(s) de document de la (des) demande(s) initiale(s) en application de l'article 76 CBE:
**00402375.0 / 1 084 696**

(71) Demandeur: **L'OREAL**
**75008 Paris (FR)**

(72) Inventeur: **Dupuis, Christine**
**75018 Paris (FR)**

(74) Mandataire: **Bourdeau, Françoise**
**L'OREAL,**
**Département Propriété Industrielle,**
**6, rue Bertrand Sincholle**
**92585 Clichy Cedex (FR)**

Remarques:
Cette demande a été déposée le 27 - 03 - 2003 comme demande divisionnaire de la demande mentionnée sous le code INID 62.

(54) **Composition cosmétique comprenant au moins un copolymère silicone/acrylate et au moins un agent photoprotecteur**

(57) L'invention a pour objet une composition cosmétique capillaire, comprenant au moins un copolymère silicone/acrylate particulier et au moins un agent photoprotecteur. Elle vise également un procédé cosmétique, en particulier un procédé de fixation et/ou de maintien de la coiffure mettant en oeuvre ladite composition ainsi que l'utilisation de cette composition pour la fabrication d'une formulation cosmétique, destinée notamment au maintien et/ou à la mise en forme de la coiffure.



Printed by Jouve, 75001 PARIS (FR)

## Description

L'invention a pour objet une composition cosmétique, comprenant au moins un copolymère silicone/acrylate particulier et au moins un agent photoprotecteur. Elle vise également un procédé cosmétique, en particulier un procédé de fixation et/ou de maintien de la coiffure mettant en oeuvre ladite composition ainsi que l'utilisation de cette composition pour la fabrication d'une formulation cosmétique, destinée notamment au maintien et/ou à la mise en forme de la coiffure.

Selon l'invention, on entend désigner de manière générale par agent photoprotecteur, tout composé ou toute association de composés, connu(s)en soi qui, par des mécanismes, également connus en soi, d'absorption et/ou de réflexion et/ou diffusion du rayonnement UV-A et/ou UV-B, permet d'empêcher, ou du moins limiter, la mise en contact dudit rayonnement avec la surface des cheveux sur laquelle ce ou ces composés ont été appliqués. En d'autres termes, ces composés peuvent être des filtres organiques photoprotecteurs absorbeurs d'UV ou des (nano-)pigments minéraux diffuseurs et/ou réflecteurs d'UV, ainsi que leurs mélanges.

Parmi les produits capillaires, notamment ceux destinés à la mise en forme et/ou au maintien de la coiffure les plus répandus sur le marché de la cosmétique, on distingue des compositions à pulvériser essentiellement constituées d'une solution le plus souvent alcoolique ou aqueuse et d'un ou plusieurs matériaux.

Lorsque les compositions sont destinées à la fixation et/ou au maintien de la coiffure, ces matériaux sont généralement des résines polymères, dont la fonction est de former des soudures entre les cheveux, appelés encore matériaux fixants, en mélange avec divers adjuvants cosmétiques. Ces compositions sont généralement conditionnées soit dans un récipient aérosol approprié mis sous pression à l'aide d'un propulseur, soit dans un flacon pompe.

On connaît également les gels ou les mousses capillaires qui sont généralement appliqués sur les cheveux mouillés avant de faire un brushing ou une mise en plis. Pour mettre en forme et fixer la coiffure, on effectue ensuite un brushing ou un séchage. Ces gels ou mousses peuvent également contenir des résines polymères.

On sait que les radiations lumineuses de longueurs d'onde comprises entre 280 nm et 400 nm altèrent les cheveux en cas d'exposition trop longue d'une personne à ces rayonnements. En particulier, les rayons de longueurs d'onde comprises entre 280 nm et 320 nm, connus sous la dénomination d'UV-B, fragilisent les cheveux et en dégradant ses différents constituants, en particulier la kératine.

Les rayons UV-A, de longueurs d'onde comprises entre 320 nm et 400 nm sont, eux, susceptibles de modifier la couleur naturelle des cheveux, notamment dans le cas de cheveux de couleur claire. Les rayons UV-A provoquent, aussi, une perte d'élasticité des cheveux qui deviennent secs et cassants, surtout au niveau des pointes.

Il a déjà été proposé des compositions capillaires comprenant des agents photoprotecteurs capables de filtrer les rayons UV-A et UV-B. Toutefois, ces compositions capillaires ne donnent pas encore entière satisfaction, car une trop faible proportion d'agents photoprotecteurs se fixent sur les cheveux. Par conséquent, la protection véritablement obtenue, après application de la composition capillaire sur les cheveux, est loin d'être égale la protection escomptée.

De surcroît, les agents photoprotecteurs qui se sont initialement fixés sur les cheveux n'y restent pas suffisament longtemps pour assurer à la personne une protection convenable et durable. Les cheveux sont donc soumis au rayonnement solaire naturel, c'est-à-dire à un rayonnement contenant des UV-A et UV-B nocifs, peu de temps déjà après l'application de la composition capillaire sur les cheveux.

Il existe donc un besoin de trouver des compositions cosmétiques, notamment pour le maintien et /ou la fixation de la coiffure, qui ne présentent pas l'ensemble des inconvénients indiqués ci-dessus, et qui en particulier fixent bien la coiffure, tout en filtrant suffisamment les rayonnement solaires et en procurant de bonnes propriétés cosmétiques.

De manière surprenante et inattendue, la Demanderesse a découvert que lorsque l'on associe certains copolymères silicone/acrylate avec des agents photoprotecteurs, il est possible d'obtenir des compositions cosmétiques répondant aux exigences exprimées ci-dessus.

L'invention a pour objet une composition cosmétique comprenant, dans un milieu cosmétiquement acceptable, au moins un copolymère silicone/acrylate et au moins un agent photoprotecteur, le copolymère silicone/acrylate étant obtenu par polymérisation radicalaire d'au moins un monomère éthyléniquement insaturé (a) en présence d'au moins un dérivé siliconé (b) comprenant des groupements oxyalkylénés.

Un autre objet de l'invention concerne un procédé cosmétique capillaire, en particulier un procédé de fixation et/ou de maintien de la coiffure mettant en oeuvre ladite composition.

Encore un autre objet de l'invention concerne l'utilisation de cette composition pouf la fabrication d'une formulation cosmétique capillaire, destinée notamment au maintien et/ou à la mise en forme de la coiffure.

Les copolymères silicone/acrylate particulièrement visés par la présente invention sont ceux décrits dans la demande internationale WO99/04750. En particulier, on préfère le copolymère commercialisé par BASF sous la dénomination Luviflex Silk. Ce polymère un copolymère greffé acrylate de tertiobutyle/ acide méthacrylique et silicone copolyol.

De préférence, le monomère (a) répond à la formule ($I_a$) :

$$X\text{-}C\text{-}CR^7{=}CHR^6 \qquad (I_a)$$

II

O

dans laquelle :

- X est choisi dans le groupe comprenant OH, OM, $OR^8$, $NH_2$, $NHR^8$, $N(R^8)_2$ ;
- M est un cation choisi parmi $Na^+$, $K^+$, $Mg^{++}$, $NH^{4+}$, alkylammonium, dialkylammonium, trialkylammonium, et tétraalkylammonium ;
- les radicaux $R^8$ peuvent être identiques ou différents et sont choisis dans le groupe comprenant l'hydrogène, les groupements alkyls linéaires ou ramifiés en Ci à $C_{40}$, les groupements alkyls en $C_1$ à $C_{40}$ mono- ou polyhydroxylés, éventuellement substitués par un ou plusieurs groupement(s) alcoxy, amino ou carboxy, les groupements polyéthers hydroxylés, les groupements N,N-diméthylaminoéthyl, 2-hydroxyéthyl, le 2-méthoxyéthyl, 2-éthoxyéthyl, hydroxypropyl, méthoxypropyl et éthoxypropyl ;
- $R^7$ et $R^6$ sont choisis indépendamment l'un de l'autre dans le groupe comprenant l'hydrogène, les groupements alkyls linéaires ou ramifiés en $C_1$ à $C_8$, méthoxy, éthoxy, 2-hydroxyéthoxy, 2-méthoxyéthoxy et 2-éthoxyéthyl, les groupements CN, COOH et COOM.

En particulier, les monomères (a) de formule (Ia) sont l'acide acrylique et ses sels, esters ou amides. Les esters peuvent être dérivés d'alkyles linéaires en $C_1$ à $C_{40}$, d'alkyles ramifiés en $C_3$ à $C_{40}$ ou d'alcools carboxyliques en $C_3$ à $C_{40}$, d'alcools polyfonctionnels ayant 2 à 8 hydroxyles, tel que l'éthylène glycol, l'hexylène glycol, le glycérol et le 1,2,6-hexanetriol, d'aminoalcools ou d'éthers d'alcools tels que le méthoxyméthanol et l'éthoxyéthanol ou les polyalkylènes glycols.

Les monomères (a) de formule (Ia) peuvent également être choisis parmi les N,N-dialkylaminoalkyl acrylates et méthacrylates et N-dialkylaminoalkylacryl- et-méthacrylamides, le groupement amide pouvant être non substitué, N-aklyl- ou N-alkylamino-monosubstitué ou N,N-dialkylamino-disubstitué, les groupes alkyles ou alkylamino étant dérivés d'unités carboxyliques linéaires en $C_1$ à $C_{40}$ ou ramifiées en $C_3$ à $C_{40}$.

Les monomères (a) de formule (Ia) pouvant être utilisés peuvent aussi être des composés substitués dérivés de l'acide acrylique ou ses sels, esters ou amides. On peut citer par exemple les acides méthacrylique, éthacrylique et 3-cyanoacrylique.

D'autres monomères (a) convenant particulièrement bien sont les esters de vinyle et d'allyle en $C_1$ à $C_{40}$, les acides carboxyliques, linéaires en $C_3$ à $C_{40}$, ou carboxycycliques en $C_3$ à $C_{40}$, les halides de vinyle ou d'allyle, les vinyllactames, de préférence la vinylpyrrolidone et le vinylcaprolactame, et les composés hétérocycles substitués par des groupement vinyles ou allyles, de préférence la vinylpyridine, la vinyloxazoline et l'allylpyridine, les N-vinylimidazoles, les diallylamines, le chlorure de vinylidène, les composés insaturés carbonés, comme le styrène ou l'isoprène, les dérivés quaternisés par l'épichlorhydrine de l'acide acrylique ou méthacrylique.

Comme autre monomère (a), on peut enfin citer les N-vinylimidazoles, les diallylamines, le chlorure de vinylidène, les composés insaturés carbonés comme le styrène et l'isoprène, les dérivés quaternisés par l'épichlorohydrine de l'acide acrylique ou méthacrylique.

On préfère tout particulièrement, comme monomère (a) l'acide acrylique, méthacrylique et éthylacrylique; le méthyl, éthyl, propyl, n-butyl, isobutyl, t-butyl, 2-éthylhexyl et décyl acrylate; le méthyl, éthyl, propyl, n-butyl, isobutyl, t-butyl, 2-éthylhexyl et décyl méthacrylate ; le méthyl, éthyl, propyl, n-butyl, isobutyl, t-butyl, 2-éthylhexyl et décyl éthacrylate; le 2,3-dihydroxypropyl acrylate ; le 2,3-dihydroxypropyl méthacrylate ; le 2-dihydroxyéthyl acrylate ; l'hydroxypropyl acrylate ; le 2-hydroxyéthyl méthacrylate; le 2-hydroxyéthyl éthacrylate ; le 2-méthoxyéthyl acrylate ; le 2-éthoxyéthyl méthacrylate ; le 2-éthoxyéthyl éthacrylate ; l'hydroxypropyl méthacrylate ; le glycéryl monoacrylate ; le glycéryl monométhacrylate ; les polyalkylènes glycols( méth-) acrylate, les acides sulfoniques insaturés, l'acrylamide, la méthacrylamide, l'éthacrylamide, la N,N-diméthylacrylamide, la N-éthylacrylamide, la N-éthylméthacrylamide, 1-vinylimidazole, N,N-diméthylaminoéthyl(méth-)acrylate, l'acide maléique, l'acide fumarique, l'anhydride maléique et ses monoesters, l'acide crotonique, l'acide itaconique, les éthers de vinyle, le vinylformamide, la vinylamine, la vinylpyridine,

la vinylimidazole, le vinylfurane, le styrène, le sulfonate de styrène, l'alcool allylique et leurs mélanges.

Le monomère (a) peut contenir également des atomes de silicium, de fluor ou encore des groupements thio. Les monomères (a) peuvent être neutralisés s'ils contiennent des groupement acides, et ceci avant ou après la polymérisation, totalement ou partiellement, de façon à ajuster la solubilité ou le degré de dispersion dans l'eau au niveau désiré. En tant qu'agent servant pour la neutralisation, on peut utiliser les bases minérales, telles que le carbonate de sodium, les bases oraganiques telles que les aminoalcools, comme les alcanolamines telles que la méthanolamine, le 2-amino-2-méthyl-1-propanol, la triéthanolamine, et les diamines comme la lysine.

Les monomères (a) peuvent être aussi quaternisés lorsqu'ils possèdent un atome d'azote basique. S'ils possèdent au moins deux doubles liaisons éthyléniques, les monomères (a) peuvent être partiellement réticulés.

Les dérivés siliconés (b) sont notamment les composés connus, sous la dénomination INCI par diméthicone copolyols ou les tensioactifs siliconés. On peut citer ceux commercialisés sous la marque Abil ® par Goldschmidt, Alkasil ® par Rhône-Poulenc, silicone Polyol Copolymer ® par Genesee, Besil ® par Wacker, Silwet ® par OSI ou Dow Corning 190® par Dow Corning.

Parmi les monomères (b), on préfère ceux qui présentent la formule I ci-après :

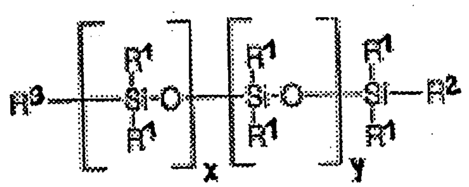

(I)

dans laquelle formule (I):

- $R^2$ est choisi parmi $CH_3$ ou le groupement

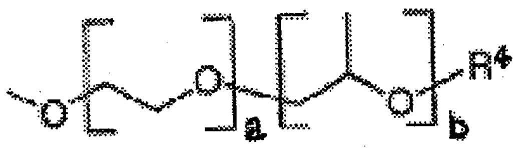

- $R^3$ est choisi parmi $CH_3$ ou le groupement $R^2$,
- $R^4$ est choisi parmi l'hydrogène, $CH_3$, ou les groupements

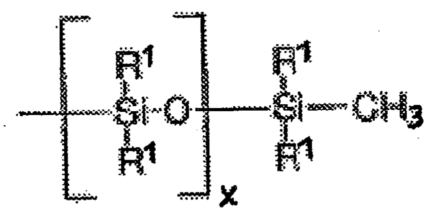

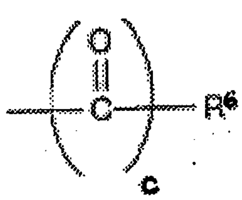

- $R^6$ est un groupement organique en $C_1$ à $C_{40}$ pouvant contenir des groupements amino, carboxyles ou sulfonates, et si c=0, $R_6$ est l'anion d'un acide inorganique ;

- les radicaux $R^1$ peuvent être identiques ou différents et sont choisis parmi les hydrocarbures aliphatiques en $C_1$ à $C_{20}$, les hydrocarbures aliphatiques ou cycloaliphatiques en $C_3$ à $C_{20}$, et les groupements $R^5$ répondant à la formule ci-après :
Parmi les dérivés siliconés (b), on préfère ceux qui présentent la formule I ci-après :

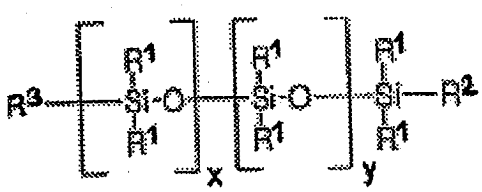

(I)

dans laquelle formule (I):

- $R^2$ est choisi parmi $CH_3$ ou le groupement

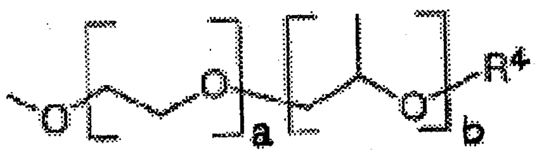

- $R^3$ est choisi parmi $CH_3$ ou le groupement $R^2$,
- $R^4$ est choisi parmi l'hydrogène, $CH_3$, ou les groupements

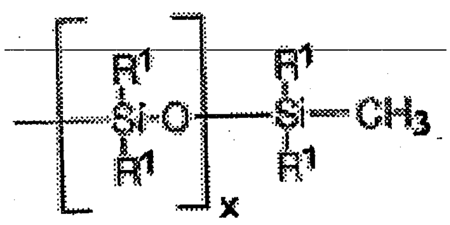

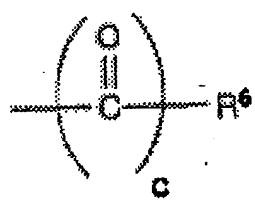

$R^6$ est un groupement organique en $C_1$ à $C_{40}$ pouvant contenir des groupements amino, carboxyles ou sulfonates, et si c=0, $R_6$ est l'anion d'un acide inorganique ;
- les radicaux $R^1$ peuvent être identiques ou différents et sont choisis parmi les hydrocarbures aliphatiques en $C_1$ à $C_{20}$, les hydrocarbures aliphatiques ou cycloaliphatiques en $C_3$ à $C_{20}$, et les groupements $R^5$ répondant à la formule ci-après :

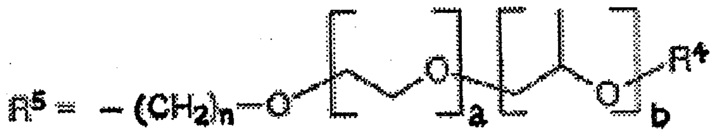

- n est un nombre entier compris entre 1 et 6,
- x et y sont des nombres entiers choisis de telle sorte que le poids moléculaire du polysiloxane soit compris entre 300 et 30 000,
  a et b sont des nombres entiers compris entre 0 et 50, et
- c est 0 ou 1.

De préférence, $R^1$ est choisi parmi les groupements méthyl, éthyl, propyl, butyl, isobutyl, pentyl, isopentyl, hexyl, octyl, décyl, dodécyl and octadécyl, les radicaux cycloaliphatiques, en particulier les groupements cyclohexyls, les groupements aromatiques, en particulier les groupements phényls ou naphthyls, les mélanges de radicaux aromatiques et aliphatiques, tels que le benzyl et le phenyléthyl, et aussi le tolyl and le xylyl.

On préfère les radicaux $R^4$ ayant pour formule -(CO)c-$R^6$, $R^6$ étant un alkyl, cycloalkyl or aryl ayant 1 à 40 atomes de carbone qui peut contenir des groupements supplémentaires tels que $NH_2$, COOH et/ou $SO_3H$.

On préfère les radicaux $R^6$ pour lesquels c=0 qui sont des phosphates ou des sulfates.

Les dérivés siliconés (b) particulièrement préférés sont ceux présentant la formule générale suivante :

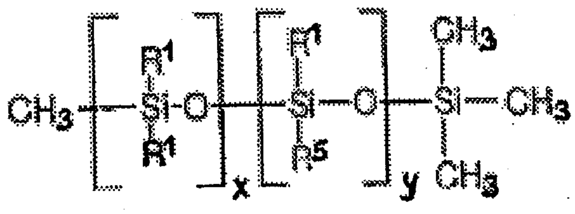

La proportion relative de dérivé (b) dans le copolymère est généralement de 0,1 à 50, et de préférence de 1 à 20 % en poids.

On préfère les copolymères silicone/acrylate solubles dans l'eau ou ceux dont la dispersibilité dans l'eau est telle que dans un mélange eau/éthanol dosé à 50 :50 en volume, ils sont solubles dans une proportion supérieure à 0,1 g/l, et de préférence supérieure à 0,2 g/l.

La composition comprend avantageusement, en pourcentage relatif en poids de la composition, de 0,1 à 20 % de ce copolymère silicone/acrylate, et de préférence, de 0,5 à 10%.

La composition comprend avantageusement, en pourcentage relatif en poids de la composition, de 0,1 à 20 % d'agent photoprotecteur, et de préférence, de 0,2 à 10%.

Parmi les filtres organiques actifs dans l'UV-A utilisables selon l'invention, on peut citer :

(1) les dérivés de dibenzoylméthane;

(2) les filtres actifs dans l'UV-A de formule (I) suivante :

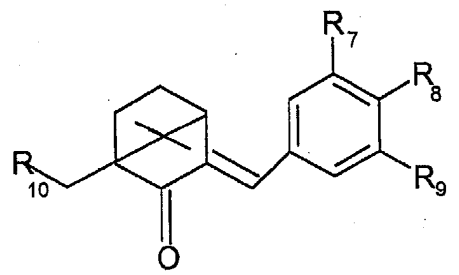

(I)

dans laquelle :

- R7 et $R_9$, identiques ou différents, désignent un hydrogène, un halogène, un radical OH, un radical alkyle, saturé ou non, linéaire ou ramifié en $C_1$-$C_{10}$; un radical alcoxy, saturé ou non, linéaire ou ramifié en $C_1$-$C_{10}$ ou un groupe $HSO_3$ ;
- $R_{10}$ désigne hydrogène ou $HSO_3$ ;
- R8 désigne hydroxy ; un groupe $OR_{11}$ où $R_{11}$ désigne un radical alkyle, saturé ou insaturé, linéaire ou ramifié en $C_1$-$C_{10}$ ; ou bien un groupe de structure suivante :

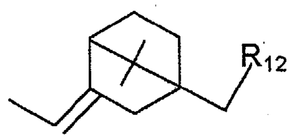

dans laquelle $R_{12}$ désigne hydrogène ou $HSO_3$ ;
ou bien un groupe de formule suivante :

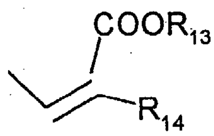

ou bien un groupe de formule suivante :

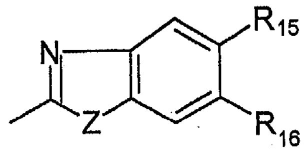

dans lesquelles :

- Z désigne un atome d'oxygène ou un radical -NH- ;
- $R_{13}$, $R_{14}$, $R_{15}$ et $R_{16}$, identiques ou différents, désignent un hydrogène, un halogène, un radical OH, un radical alkyle, saturé ou non, linéaire ou ramifié en $C_1$-$C_{10}$ ; un radical alcoxy, saturé ou non, linéaire ou ramifié en $C_1$-$C_{10}$ ou un groupe $HSO_3$ ;

(3) les filtres actifs dans l'UV-A du type benzimidazole ou benzoxazole de formule (II) suivante :

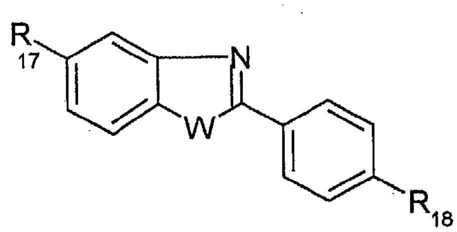

(II)

dans laquelle :

- W désigne un atome d'oxygène ou un radical -NH-,
- $R_{17}$ désigne hydrogène ou $HSO_3$ ;
- $R_{18}$ désigne un radical alcoxy, linéaire ou ramifié, contenant de 1 à 10 atomes de carbone environ ou un groupement de formule suivante :

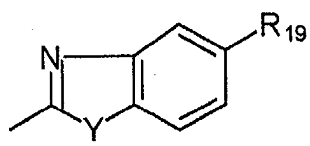

où Y représente un atome d'oxygène ou un radical -NH- et $R_{19}$ désigne hydrogène ou $HSO_3$ ;

- $R_{19}$ désigne hydrogène ou $HSO_3$ ;

(4) les dérivés de benzophénone tels que ceux de formule (III) suivante :

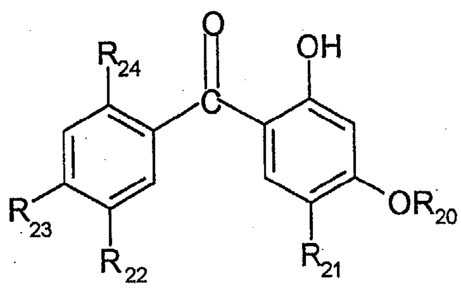

(III)

dans laquelle :

$R_{20}$ désigne hydrogène ou un radical alkyle, saturé ou non, linéaire ou ramifié en $C_1$-$C_{10}$ ;
$R_{21}$ et $R_{22}$, identiques ou différents, désignent un hydrogène, un halogène, un radical OH, un radical alkyle, saturé ou non, linéaire ou ramifié en $C_1$-$C_{10}$ ; un radical alcoxy, saturé ou non, linéaire ou ramifié en $C_1$-$C_{10}$ ou un groupe $HSO_3$ ;
$R_{23}$ désigne hydrogène ; OH ; un radical alkyle, saturé ou non, linéaire ou ramifié en $C_1$-$C_{10}$ ou un radical alcoxy, saturé ou non, linéaire ou ramifié en $C_1$-$C_{10}$ ;
$R_{24}$ désigne OH, un atome d'hydrogène ou un radical alkyle, saturé ou non, linéaire ou ramifié en $C_1$-$C_{10}$ ;

(5) les dérivés silanes ou les polyorganosiloxanes à groupement benzophénone tels que ceux décrits dans les documents EP-A-0389 377, FR-A-2 657 351 et EP-A-0 655 453 ;

(6) les anthranilates ;

(7) les benzotriazoles et silicones benzotriazoles ;

(8) leurs mélanges.

En tant que benzotriazoles ou dérivé de benzotriazole, on utilise de préférence ceux de formule (I) suivante :

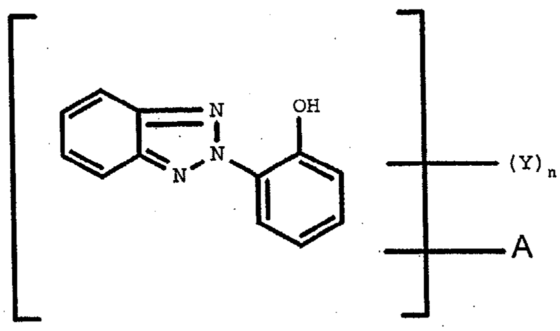

(I)

dans laquelle :

- A désigne hydrogène ou un radical divalent -L-W-
- Y, identiques ou différents, sont choisis parmi les radicaux alkyles en $C_1$-$C_{10}$, les halogènes, les radicaux alkoxy en $C_1$-$C_{10}$, des groupes sulfoniques, étant entendu que, dans ce dernier cas, deux Y adjacents d'un même noyau aromatique peuvent former ensemble un groupement alkylidène dioxy dans lequel le groupe alkylidène contient de 1 à 2 atomes de carbone ; sous réserve que les radicaux Y soient différents d'un groupe sulfonique quand A est différent d'hydrogène ;
- n vaut 1, 2 ou 3 ;
- L est un radical divalent de formule (II) suivante :

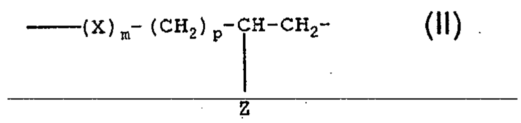

(II)

dans laquelle :

- X représente O ou NH,
- Z représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement.
- W est un radical de formule (1), (2) ou (3) suivante :

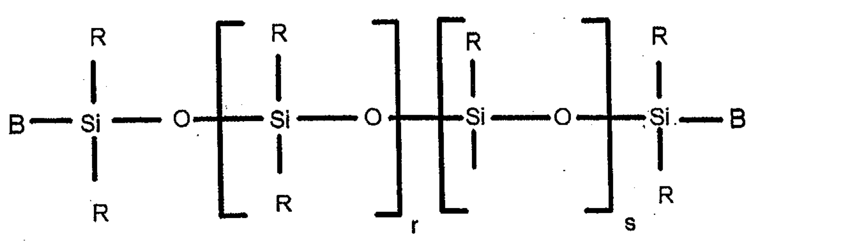

(1)

ou

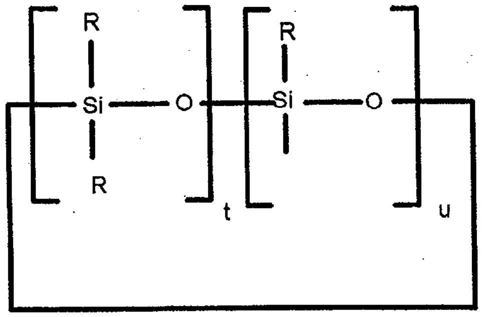

(2)

ou

$$-Si(R)_3 \qquad (3)$$

dans lesquelles :

- R, identiques ou différents, sont choisis parmi les radicaux alkyles en $C_1$-$C_{10}$, phényle et trifluoro-3,3,3 propyle, au moins 80% en nombre des radicaux R étant méthyle,
- B, identiques ou différents, sont choisis parmi les radicaux R et le radical V de formule suivante :

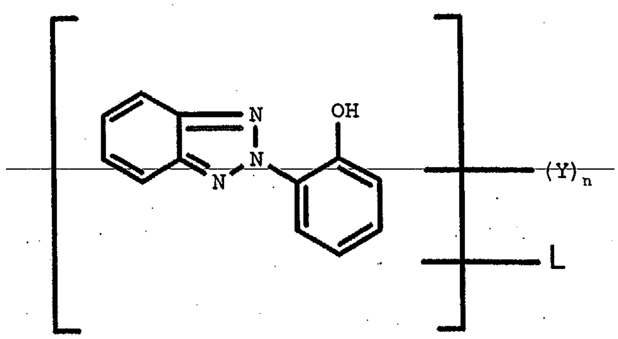

dans laquelle Y, n et L ont les mêmes significations indiquées ci-dessus ;

- r est un nombre entier compris entre 0 et 50 inclusivement, et s est un nombre entier compris entre 1 et 20 inclusivement, et si s=0 au moins l'un des deux radicaux B désigne V ;
- u est un nombre entier compris entre 1 et 6 inclusivement, et t est un nombre entier compris entre 0 et 10 inclusivement, étant entendu que t + u est égal ou supérieur à 3.

Les dérivés de benzotriazole de formule (I) sont des filtres déjà connus en soi. Ils sont décrits et préparés selon les synthèses indiquées dans les brevets US-4316033 et US-4328346 ; EP-B-0354145 et EP-B-0392883, EP-B-0660701 (faisant partie intégrante du contenu de la description).

Parmi les dérivés du dibenzoylméthane rentrant particulièrement bien dans le cadre de la présente invention, on peut notamment citer, de manière non limitative :

- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4,4'-diméthoxydibenzoylméthane
- le 4-tert.-butyl-4'-méthoxydibenzoylméthane

- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane

Parmi les dérivés du dibenzoylméthane mentionnés ci-dessus, on préfère tout particulièrement, selon la présente invention, mettre en oeuvre le 4-(ter.-butyl) 4'-méthoxy dibenzoylméthane, notamment celui proposé à la vente sous la dénomination commerciale de "PARSOL 1789" par la Société GIVAUDAN, ce filtre répondant donc à la formule développée suivante :

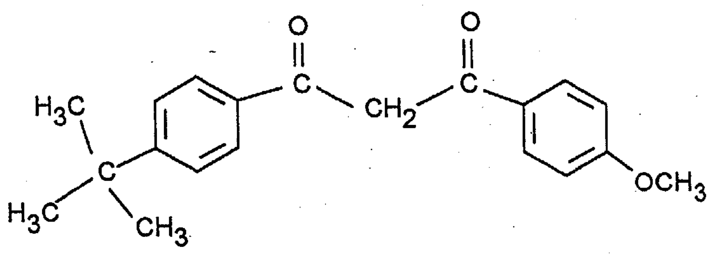

Un autre dérivé du dibenzoylméthane préféré selon la présente invention est le 4-isopropyl-dibenzoylméthane, filtre vendu sous la dénomination de "EUSOLEX 8020" par la Société MERCK, et répondant à la formule développée suivante :

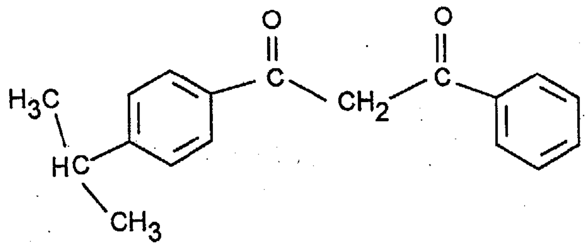

Les composés de structures (I) ci-dessus sont respectivement décrits dans le brevet US 4 585 597 et les demandes de brevets FR 2 236 515, 2 282 426, 2 645 148, 2 430 938 et 2 592 380.

Un composé de formule (I) particulièrement préféré est l'acide benzène 1,4-[di(3-méthylidènecampho 10-sulfonique)] tel que le produit vendu sous le nom MESORYL SX par le société CHIMEX.

L' acide benzène 1,4-di(3-méthylidène-10-camphosulfonique) et ses différents sels (composé D), décrits notamment dans les demandes de brevets FR-A- 2 528 420 et FR-A- 2 639 347, sont des filtres déjà connus en soi (filtres dits à large bande) capables en effet d'absorber les rayons ultraviolets de longueur d'ondes comprises entre 280 et 400 nm, avec des maxima d'absorption compris entre 320 et 400 nm, en particulier aux alentours de 345 nm. Ces filtres répondent à la formule générale suivante :

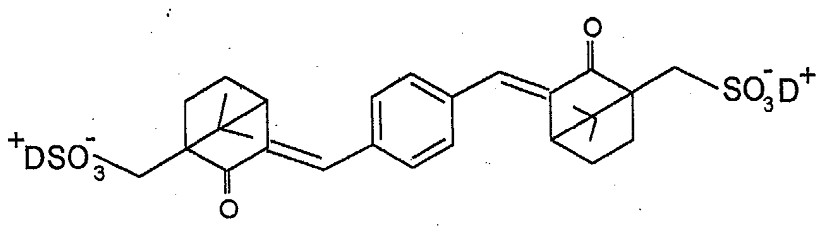

dans laquelle D désigne un atome d'hydrogène, un métal alcalin ou encore un radical $NH(R_{25})_3+$ dans lequel les radicaux $R_{25}$, qui peuvent être identiques ou différents, désignent un atome d'hydrogène, un radical alkyle ou hydroxyalkyle en $C_1$-$C_4$ ou encore un groupement $Mn^+/n$, $Mn^+$ désignant un cation métallique polyvalent dans lequel n est est égal à 2 ou 3 ou 4, $M^{n+}$ désignant de préférence un cation métallique choisi parmi $Ca^{2+}$, $Zn^{2+}$, $Mg^{2+}$, $Ba^{2+}$, $Al^{3+}$

et $Zr^{4+}$. Il est bien entendu que les composés de formule (I) ci-dessus peuvent donner lieu à l'isomère "cis-trans" autour d'une ou plusieurs double(s) liaison(s) et que tous les isomères rentrent dans le cadre de la présente invention.

On peut citer, comme exemples de composés benzimidazole particuliers répondant à la formule (II),:

- l'acide benzène 1,4 -di(benzimidazol -2 yl-5-sulfonique).
- l'acide benzène 1,4-di (benzoxazol -2 yl -5-sulfonique) ; ainsi que leurs formes partiellement ou totalement neutralisées.

Parmi les dérivés de benzophénone de formule (III), on peut citer plus particulièrement ceux choisis dans le groupe constitué par :

- la 2,4-dihydroxybenzophénone (benzophénone-1), comme le produit vendu sous le nom UVINUL 400 par BASF ;
- la 2,2',4,4'-tétra-hydroxybenzophénone (benzophénone-2), comme le produit vendu sous le nom UVINUL D50 par BASF ;
- la 2-hydroxy-4-méthoxy-benzophénone, encore appelé oxybenzone (benzophénone-3) comme le produit vendu sous le nom UVINUL M40 par BASF ;
- l'acide 2-hydroxy-4-méthoxy-benzophénone-5-sulfonique, encore appelé sulisobenzone (benzophénone-4) comme le produit vendu sous le nom UVINUL MS 40 par BASF ; ainsi que sa forme sulfonate de sodium (benzophénone-5) ;
- la 2,2'-dihydroxy-4,4'-diméthoxy-benzophénone (benzophénone-6) comme le produit vendu sous le nom HELISORB 11 par NORQUAY ;
- la 5-chloro-2-hydroxybenzophénone (benzophénone-7) ;
- la 2,2'-dihydroxy-4-méthoxy-benzophénone, encore appelé dioxybenzone ou benzophénone-8, comme le produit vendu sous le nom SPECTRA-SORB UV-24 par AMERICAN CYANAMID ;
- le sel disodique du diacide 2,2'-dihydroxy-4,4'-diméthoxy-benzophénone-5,5'-disulfonique ou benzophénone-9 comme le produit vendu sous le nom UVINUL DS49 par BASF ;
- la 2-hydroxy-4-méthoxy-4'-méthyl-benzophénone (benzophénone-10);
- la benzophénone-11 comme le produit vendu sous le nom UVINUL M493 par BASF ;
- la 2-hydroxy-4-(octyloxy)benzophénone (benzophénone-12).

Parmi les anthranilates utilisables selon l'invention, on peut citer tout particulièrement l'anthranilate de menthyle tel que le produit vendu sous le nom NEO HELIOPAN MA par la société Haarmann & Reimer.

En tant que filtres UV-B particulièrement utilisés dans le cadre de la présente invention, on peut citer les dérivés de triazines décrits notamment dans le brevet US4617390 et les demandes de brevet EP-A-087098, EP-A-0517104, EP-A-0570838 et EP-A-0796851. On connaît notamment la 2,4,6-tris[p-(2'-éthylhexyl-1'-oxy-carbonyl)anilino]-1,3,5-triazine vendue notamment sous la dénomination commerciale de "UVINUL T 150" par la Société BASF.

Avantageusement, on utilise également les dérivés de l'acide cinnamique tels que le 4-méthoxy cinnamate d'isopentyle, le 4-méthoxy cinnamate de 2-éthylhexyle, le diisopropyl cinnamate de méthyle, le 4-méthoxy cinnamate d'isoamyle, le 4-méthoxy cinnamate de diéthanolamine.

Parmi les dérivés de l'acide cinnamique mentionnés ci-dessus, on préfère tout particulièrement, selon la présente invention, mettre en oeuvre le p-méthoxycinnamate de 2-éthylhexyle, vendu notamment sous le nom commercial «PARSOL MCX » par la Société GIVAUDAN.

On peut également utiliser les 2 cyano-33-diphénylacrylate d'alkyle et, de manière préférentielle, l'octocrylidène vendu sous le nom Uvinul N 539 parla Société BASF.

En tant qu'agent photoprotecteur, on peut encore avantageusement utiliser les paraaminobenzoates ou paradiméthylaminobenzoates d'alkyle ou éthyle, propyle ou octyle de glycérine. On peut aussi utiliser le 3,4'-méthylbenzylidène camphre, le 3-benzylidène camphre, et le méthylsulfate de 3,4'- triméthylammonium de benzylidène camphre et l'acide 3-(4'-sulfobenzylidène) camphre et ses sels.

En tant qu'agent photoprotecteur, on préfère tout-particulièrement le p-méthoxycinnamate de 2-éthylehexyle, les dérivés de triazines et, en particulier le 2,4,6 tris-p(2-éthylhexyl-1-acycarbonyl) anilino 1,3,5-triazine, les salicylates, et en particulier les salicylates de méthyle ou d'octyle, les para-aminobenzoates ou paradiméthylaminobenzoates, le 2-cyano-3,3 phénylacrylate d'alkyle, les nanopigments d'oxydes métalliques ou leurs mélanges.

Les compositions cosmétiques selon l'invention peuvent encore contenir, en tant qu'agent photoprotecteurs, des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A-0518772 et EP-A-0518773.

Le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en $C_1$-$C_4$.

Parmi ces alcools, on peut citer l'éthanol, l'isopropanol. L'éthanol est particulièrement préféré.

La composition de l'invention peut également contenir au moins un additif choisi parmi les tensioactifs anioniques, cationiques, non ioniques ou amphotères, les parfums, les filtres, les conservateurs, les protéines, les vitamines, les polymères autres que ceux de l'invention, les huiles végétales, minérales ou synthétiques et tout autre additif classiquement utilisé dans les compositions cosmétiques.

Bien entendu, l'homme de l'art veillera à choisir le ou les éventuels composés à ajouter à la -composition selon l'invention de manière telle que les propriétés avantageuses attachées intrinsèquement à la composition conforme à l'invention ne soient pas, ou substantiellement pas, altérées par l'addition envisagée.

Ces compositions peuvent être conditionnées sous diverses formes, notamment dans des flacons pompes ou dans des récipients aérosols, afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux. Les compositions conformes à l'invention peuvent aussi se présenter sous la forme de crèmes, de gels, d'émulsions, de lotions ou de cires.

Lorsque la composition selon l'invention est conditionnée sous forme d'aérosol en vue d'obtenir une laque ou une mousse, elle comprend au moins un agent propulseur qui peut être choisi parmi les hydrocarbures volatils tels que le n-butane, le propane, l'isobutane, le pentane, un hydrocarbure halogène et leurs mélanges. On peut également utiliser en tant qu'agent propulseur le gaz carbonique, le protoxyde d'azote, le

diméthyléther (DME), l'azote, l'air comprimé. On peut aussi utiliser des mélanges de propulseurs. De préférence, on utilise le diméthyl éther.

Avantageusement, l'agent propulseur est présent à une concentration comprise entre 5 et 90 % en poids par rapport au poids total de la composition dans le dispositif aérosol et, plus particulièrement, à une concentration comprise entre 10 et 60 %.

Les compositions conformes à l'invention sont particulièrement adaptés pour des cheveux secs ou humides, en tant que produits de coiffage.

Les compositions conformes à l'inventions peuvent être appliquées sur la peau, les ongles, les lèvres, les cheveux, les sourcils et les cils.

## Revendications

1. Composition cosmétique comprenant, dans un milieu cosmétiquement acceptable, au moins un copolymère silicone/acrylate et au moins un agent photoprotecteur, **caractérisée par le fait que** le copolymère silicone/acrylate est obtenu par polymérisation radicalaire d'au moins un monomère éthyléniquement insaturé (a) en présence d'au moins un dérivé siliconé (b) comprenant des groupements oxyalkylénés, et l'agent photoprotecteur est choisi parmi :

   (1) les dérivés de dibenzoylméthane ;

   (2) les filtres actifs dans l'UV-A de formule (I) suivante :

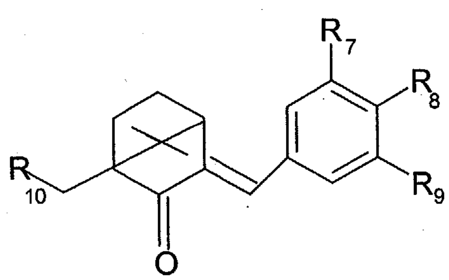

(I)

   dans laquelle :

   - $R_7$ et $R_9$, identiques ou différents, désignent un hydrogène, un halogène, un radical OH, un radical alkyle,

saturé ou non, linéaire ou ramifié en $C_1$-$C_{10}$ ; un radical alcoxy, saturé ou non, linéaire ou ramifié en $C_1$-$C_{10}$ ou un groupe $HSO_3$ ;

- $R_{10}$ désigne hydrogène ou $HSO_3$ ;
- R8 désigne hydroxy ; un groupe $OR_{11}$ où $R_{11}$ désigne un radical alkyle, saturé

ou insaturé, linéaire ou ramifié en $C_1$-$C_{10}$ ; ou bien un groupe de structure suivante :

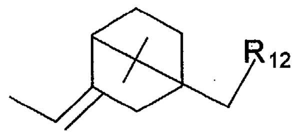

dans laquelle $R_{12}$ désigne hydrogène ou $HSO_3$ ;
ou bien un groupe de formule suivante :

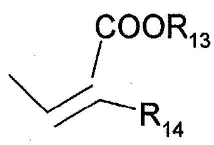

ou bien un groupe de formule suivante :

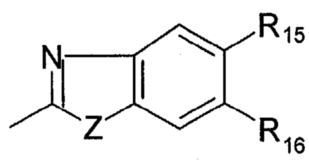

dans lesquelles :

- Z désigne un atome d'oxygène ou un radical -NH- ;
- $R_{13}$, $R_{14}$, $R_{15}$ et $R_{16}$, identiques ou différents, désignent un hydrogène, un halogène, un radical OH, un radical alkyle, saturé ou non, linéaire ou ramifié en $C_1$-$C_{10}$ ; un radical alcoxy, saturé ou non, linéaire ou ramifié en $C_1$-$C_{10}$ ou un groupe $HSO_3$ ;

(3) les filtres actifs dans l'UV-A du type benzimidazole ou benzoxazole de formule (II) suivante :

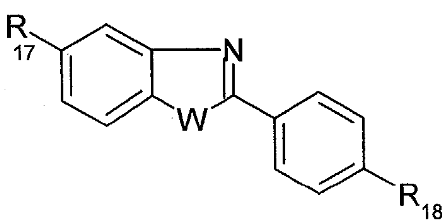

(II)

dans laquelle :

- W désigne un atome d'oxygène ou un radical -NH-,
- $R_{17}$ désigne hydrogène ou $HSO_3$ ;
- $R_{18}$ désigne un radical alcoxy, linéaire ou ramifié, contenant de 1 à 10 atomes de carbone environ ou un

groupement de formule suivante :

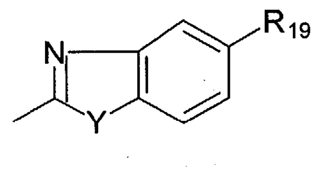

où Y représente un atome d'oxygène ou un radical -NH- et $R_{19}$ désigne hydrogène ou $HSO_3$ ;

- $R_{19}$ désigne hydrogène ou $HSO_3$ ;

(4) les dérivés silanes ou les polyorganosiloxanes à groupement benzophénone ;

(5) les anthranilates ;

(6) les benzotriazoles et silicones benzotriazoles ;

(7) leurs mélanges;

(8) les dérivés de triazines et, en particulier le 2,4,6 tris-p(2-éthylhexyl-1-acycarbonyl) anilino 1,3,5-triazine, les salicylates , et en particulier les salycilates de méthyle ou d'octyle, les para-aminobenzoates ou paradiméthylaminobenzoates, le 2-cyano-3,3 phénylacrylate d'alkyle, les nanopigments d'oxydes métalliques ou leurs mélanges;

(9) le 3,4'-méthylbenzylidène camphre, le 3-benzylidène camphre, et le méthylsulfate de 3,4'- triméthylammonium de benzylidène camphre et l'acide 3-(4'-sulfobenzylidène) camphre et ses sels.

**2.** Composition selon la revendication 1, **caractérisée par le fait que** le monomère (a) répond à la formule ($I_a$) :

$$X\text{-}C\text{-}CR^7{=}CHR^6 \qquad (I_a)$$
II
O

dans laquelle :

- X est choisi dans le groupe comprenant OH, OM, $OR^8$, $NH_2$, $NHR^8$, $N(R^8)2$;
- M est un cation choisi parmi $Na^+$, $K^+$, $Mg^{++}$, $NH^{4+}$, alkylammonium, dialkylammonium, trialkylammonium, et tétraalkylammonium ;
- les radicaux $R^8$ peuvent être identiques ou différents et sont choisis dans le groupe comprenant l'hydrogène, les groupements alkyls linéaires ou ramifiés en $C_1$ à $C_{40}$, les groupements alkyls en $C_1$ à $C_{40}$ mono- ou polyhydroxylés, éventuellement substitués par un ou plusieurs groupement(s) alcoxy, amino ou carboxy, les groupements polyéthers hydroxylés, les groupements N,N-diméthylaminoéthyl, 2-hydroxyéthyl, 2-méthoxyéthyl, 2-éthoxyéthyl, hydroxypropyl, méthoxypropyl et éthoxypropyl ;
- $R^7$ et $R^6$ sont choisis indépendamment l'un de l'autre dans le groupe comprenant l'hydrogène, les groupements alkyls linéaires ou ramifiés en $C_1$ à $C_8$, méthoxy, éthoxy, 2-hydroxyéthoxy, 2-méthoxyéthoxy et 2-éthoxyéthyl, les groupements CN, COOH et COOM.

**3.** Composition cosmétique selon la revendication 1, **caractérisée par le fait que** le dérivé siliconé (b) répond à la formule I ci-après :

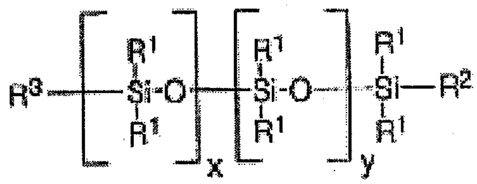 (I)

dans laquelle formule (I):

- $R^2$ est choisi parmi $CH_3$ ou le groupement

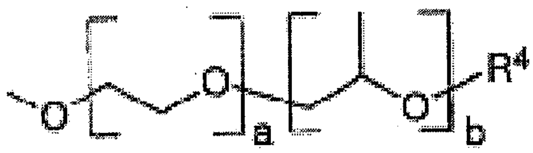

- $R^3$ est choisi parmi $CH_3$ ou le groupement $R^2$,
- $R^4$ est choisi parmi l'hydrogène, $CH_3$, ou les groupements

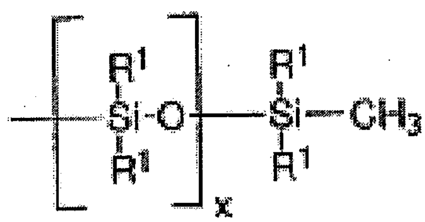

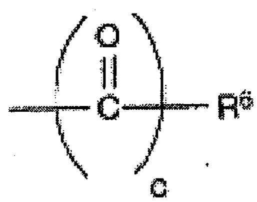

$R^6$ est un groupement organique en $C_1$ à $C_{40}$ pouvant contenir des groupements amino, carboxyles ou sulfonates, et si c=0, $R_6$ est l'anion d'un acide inorganique ;

- les radicaux $R^1$ peuvent être identiques ou différents et sont choisis parmi les hydrocarbures aliphatiques en Ci à $C_{20}$, les hydrocarbures aliphatiques ou cycloaliphatiques en $C_3$ à $C_{20}$, et les groupements $R^5$ répondant à la formule ci-après :

$R^5$ = 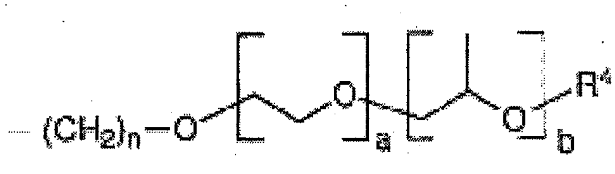

- n est un nombre entier compris entre 1 et 6,

- x et y sont des nombres entiers choisis de telle sorte que le poids moléculaire du polysiloxane soit compris entre 300 et 30 000,
- a et b sont des nombres entiers compris entre 0 et 50, et
- c est 0 ou 1.

4. Composition selon la revendication 1, **caractérisée par le fait que** le monomère

(a) est choisi parmi les esters de vinyle et d'allyle en $C_1$ à $C_{40}$, les acides carboxyliques, linéaires en $C_3$ à $C_{40}$, ou carboxycycliques en $C_3$ à $C_{40}$, les halides de vinyle ou d'allyle, les vinyllactames, de préférence la vinylpyrrolidone et le vinylcaprolactame, les composés hétérocycles substitués par des groupement vinyls ou allyls, de préférence la vinylpyridine, la vinyloxazoline et l'allylpyridine, les N-vinylimidazoles, les diallylamines, le chlorure de vinylidène, les composés insaturés carbonés, comme le styrène ou l'isoprène, les dérivés quaternisés par l'épichlorhydrine de l'acide acrylique

ou méthacrylique.

5. Composition selon la revendication 1, **caractérisée par le fait que** le monomère (a) est choisi dans le groupe comprenant l'acide acrylique, méthacrylique et éthylacrylique ; le méthyl, éthyl, propyl, n-butyl, isobutyl, t-butyl, 2-éthylhexyl et décyl acrylate ; le méthyl, éthyl, propyl, n-butyl, isobutyl, t-butyl, 2-éthylhexyl et décyl méthacrylate ; le méthyl, éthyl, propyl, n-butyl, isobutyl, t-butyl, 2-éthylhexyl et décyl éthacrylate ; le 2,3-dihydroxypropyl acrylate ; le 2,3-dihydroxypropyl méthacrylate ; le 2-dihydroxyéthyl acrylate ; l'hydroxypropyl acrylate ; le 2-hydroxyéthyl méthacrylate ; le 2-hydroxyéthyl éthacrylate ; le 2-méthoxyéthyl acrylate ; le 2-éthoxyéthyl méthacrylate ; le 2-éthoxyéthyl éthacrylate ; l'hydroxypropyl méthacrylate ; le glycéryl monoacrylate ; le glycéryl monométhacrylate ; les polyalkylènes glycols( méth-) acrylate, les acides sulfoniques insaturés, l'acrylamide, la méthacrylamide, l'éthacrylamide, la N,N-diméthylacrylamide, la N-éthylacrylamide, la N-éthylméthacrylamide, 1-vinylimidazole, N,N-diméthylaminoéthyl(méth-)acrylate, l'acide maléique, l'acide fumarique, l'anhydride maléique et ses monoesters, l'acide crotonique, l'acide itaconique, les éthers de vinyle, le vinylformamide, la vinylamine, la vinylpyridine, la vinylimidazole, le vinylfurane, le styrène, le sulfonate de styrène, l'alcool allylique et leurs mélanges.

6. Composition selon l'une quelconque des revendications précédentes, **caractérisée par le fait que** les dérivés siliconés (b) sont choisis parmi les diméthicone copolyols ou les tensioactifs siliconés.

7. Composition selon l'une quelconque des revendications précédentes, **caractérisée par le fait qu'**elle comprend, en pourcentage relatif en poids, de 0,1 à 20 % de copolymère silicone/acrylate, et plus préférentiellement de 0,5 à 10 % de ce copolymère.

8. Composition selon l'une quelconque des revendications précédentes, **caractérisée par le fait qu'**elle contient en outre au moins un additif choisi parmi les tensioactifs anioniques, cationiques, non ioniques ou amphotères, les parfums, les conservateurs, les protéines, les vitamines, les polymères autres que ceux de l'invention, les huiles végétales, minérales ou synthétiques.

9. Procédé de maintien ou de mise en forme de la coiffure, **caractérisé par le fait qu'**il comprend la mise en oeuvre d'une composition conforme à l'une quelconque des revendications 1 à 8.

10. Utilisation d'une composition selon l'une quelconque des revendications 1 à 8, pour la fabrication d'un produit cosmétique, en particulier capillaire.

11. Utilisation selon l'une quelconque des revendications 1 à 8 pour la peau, les cheveux, les sourcils et les cils.